# EUROPÄISCHE PATENTANMELDUNG

(11) **EP 1 156 025 A2**
(43) Veröffentlichungstag der Anmeldung: **21.11.2001**
(21) Anmeldenummer: 01110940.2
(22) Anmeldetag: 05.05.2001
(51) Int. Cl.: C07B 63/00, C07C 2/86, C07C 13/40, C07C 67/343, C07C 69/675, C07F 3/06

(54) **Verwendung von Metallhydridbehandeltem Zink in der metallorganischen Synthese**

(30) Priorität: 19.05.2000 DE 10024776
(71) Anmelder: Goldschmidt AG, 45127 Essen (DE)
(72) Erfinder: Frommeyer, Georg, Prof. Dr., 40699 Erkrath (DE); Knott, Wilfried, Dr., 45141 Essen (DE); Weier, Andreas, Dr., 45289 Essen (DE); Weiss, Jurij, Dr., 44793 Bochum (DE); Windbiel, Dagmar, 45289 Essen (DE)

(57) **Zusammenfassung**

Die vorliegende Erfindung beschreibt die Verwendung von Metallhydrid-behandeltem Zink in der metallorganischen Synthese.

## Beschreibung

Die vorliegende Erfindung beschreibt die Verwendung von Metallhydrid-behandeltem Zink in der metallorganischen Synthese.

Aus der noch unveröffentlichten europäischen Anmeldung 00102815.8 ist die Umsetzung von metallischem Zink mit Metallhydriden, insbesondere Magnesium-Hydrid bekannt. Auf den Inhalt dieser Patentanmeldung wird insoweit voll umfänglich Bezug genommen.

Bisherige Anwendungsgebiete von Hydriden in der Metallurgie, bzw. den Materialwissenschaften beschränken sich hauptsächlich auf den Einsatz zur Darstellung von Metallschäumen (beispielsweise TiH₂ oder MgH₂ als Treibmittel für Aluminiumschäume; US-A-5 972 285; DE-C-41 01 630; EP-B-0 559 097), Herstellung von mikrokristallinen oder amorphen Metallen oder Legierungen durch Reduktion entsprechender Metallsalze mittels Hydriden (US-A-5 951 739; DE-C-3 934 351; Bönnemann, Brijoux, Joussen, Angew. Chem., 1990, 102, 324-326), festkörperchemische Phasenumwandlungen von Legierungen über Hydridbildung durch Behandlung mit Wasserstoff (Sakamoto, Chen, Ura, Flanagan, Ber. Bunsen-Ges. 1995, 99, 807-820), Decarburierung von Stahl mittels Hydriden (JP-C-05 287 361) und die Aktivierung von Aluminium mit Alkali- oder Erdalkalihydriden für die Synthese von Aluminiumalkylen (Houben-Weyl, Methoden der organischen Chemie, 1970, Band 13/4, 33; US-A-2 989 557; US-A-3 050 541; BE-C-559 404; US-A-3 077 490).

Metallorganische Zink-Reagenzien erschließen im Bereich der organischen Chemie wichtige Zugänge zu einer Reihe von Pharmazeutika (beispielsweise Steroide), Naturstoff-Derivaten (beispielsweise Terpene, Fettsäuren) und Insektiziden (beispielsweise Pyrethroide).

Stellvertretend für den Einsatz von Zink in metallorganischen Reaktionen sei hier beispielhaft die Anwendung in Simmons-Smith- und Reformatsky-Reaktionen beschrieben.

In Simmons-Smith-Reaktionen werden Olefine mit Dijodmethan und metallischem Zink, typischerweise in Diethylether als Lösungsmittel zu Cyclopropanen umgesetzt. Als reaktive Zwischenspezies bildet sich nach Literatur (Houben-Weyl, Methoden der organischen Chemie, 1973, Band 13/2a, 838-852) aus CH₂I₂ und Zn zunächst das Insertionsprodukt ICH₂ZnI, das im Folgeschritt unter Abspaltung von ZnI₂ das Carben CH₂ auf die Doppelbindung des eingesetzten Olefins in einem Ein-Stufen-Mechanismus überträgt.

In Reformatsky-Reaktionen werden α-Halogenester mit metallischem Zink und Aldehyden oder Ketonen zu β-Hydroxycarbonsäureestern umgesetzt. Im ersten Schritt insertiert nach Literatur (Houben-Weyl, Methoden der organischen Chemie, 1973, Band 13/2a, 809-838) das Zink in die Halogen-Kohlenstoff-Bindung des α-Halogenesters unter Bildung einer nukleophilen zinkorganischen Zwischenstufe, die im zweiten Schritt mit den elektrophilen Carbonylkohlenstoff-Atomen von Aldehyden oder Ketonen zu β-Hydroxycarbonsäureestern reagiert.

In den beiden vorgestellten Reaktionstypen ist für eine möglichst hohe Produktausbeute nicht nur eine geeignete Reaktionsführung (Art des Lösungsmittels, Temperatur, Wahl der Konzentrationsverhältnisse, Reaktionsdauer etc.) sondern auch eine entsprechende Vorbehandlung bzw. Aktivierung des eingesetzten metallischen Zinks entscheidend. Zur Erhöhung der Reaktivität (Zerstörung der Oxidschicht, Erreichen einer besonders feinen Verteilung) von Zink in Simmons-Smith- und Reformatsky-Reaktionen sind bisher zahlreiche physikalische (beispielsweise mechanisches Zermahlen, Anwendung von Ultraschall, Metallverdampfung, elektrolytische Ultrahochreinigung) und chemische Methoden (beispielsweise Rieke-Verfahren, Zink-Kupfer-Paarbildung, Ätz-Verfahren, Kaliumgraphit-Verfahren) entwickelt worden (A. Fürstner, Active Metals, Preparation, Characterization, Applications, VCH, 1996).

Als Kriterium für die Qualität, bzw. Effizienz der jeweils angewandten physikalischen oder chemischen Aktivierungsmethode kann der Einsatz entsprechend behandelten metallischen Zinks in der metallorganischen Synthesechemie, beispielsweise in Simmons-Smith- und Reformatsky-Reaktionen, anhand der Bestimmung der jeweiligen Produktausbeute getestet werden.

Bereits Simmons und Smith (J. Am. Chem. Soc., 1958, 80, 5323-5324; J. Am. Chem. Soc., 1959, 81, 4256-4264) beschreiben die nach den Autoren benannte Cyclopropanierungsreaktion unter Verwendung eines Zink-Kupfer-Paares, das verhältnismäßig aufwendig durch Reduktion eines Kupferoxid-Zink-Gemisches im Wasserstoffstrom bei 500 °C erhalten wurde. Spätere Arbeiten anderer Autoren betonen immer wieder die Problematik einer guten und reproduzierbaren Präparationsmethodik für das Zink-Kupfer-Paar und zeigen alternative Herstellverfahren auf (beispielsweise Shank, Shechter, J. Org. Chem, 1959, 24, 1825-1826; LeGoff, J. Org. Chem., 1964, 29, 2048-2050). Die präparativ wohl einfachste Generierungsmethode beschreiben Rawson und Harrison (J. Org. Chem., 1970, 35, 2057-2058).

Sie erzeugen das Zink-Kupfer-Paar durch 30-minütiges Erhitzen einer Suspension von Zink mit 10 % Kupfer (I) chlorid. Neuere Arbeiten über Alternativen zum Zink-Kupfer-Paar beinhalten beispielsweise auch den Einsatz von elektrolytisch ultrahochgereinigtem Zink (Takai, Kakiuchi, Utimoto, J. Org. Chem., 1994, 59, 2671-2673), den Einsatz von mit geringen Mengen elementarem Jod aktivierten Zinks in der Steroidsynthese (DD-C-143 782) oder auch den Zusatz katalytischer Mengen komplexer Hydride wie beispielsweise Natrium-bis-(2-methoxyethoxy)aluminiumhydrid (Vitride® ) in der Cyclopropanierung ungesättigter Fettsäureester (US-A-4 472 313).

Im Falle der Anwendung von Zink in Reformatsky-Reaktionen zeigt sich eine ähnliche Art und Vielfalt der Aktivierungsmethoden wie bereits bei den Simmons-Smith-Reaktionen beschrieben. Einfaches Aktivieren durch Reagenzien wie Jod, Dibrommethan usw. oder Waschen mit verdünnten Mineralsäuren erbringt häufig nur mäßigen Erfolg. Wesentlich effektiver ist wiederum die Verwendung spezieller Legierungen (beispielsweise Zink-Kupfer- oder Zink-Silber-Paar). Ein sehr effektives chemisches Aktivierungsverfahren ist die Reduktion von Zinkhalogeniden mit Kalium nach dem Rieke-Verfahren. Darüber hinaus existieren noch weitere Verfahren, unter anderem die Reduktion mit Kaliumgraphit oder die Verwendung von Ultraschall (Fürstner, Angew. Chem., 1993, 105, 171-197; Rieke, Uhm, Synthesis, 1975, 452-453; Han, Boudjouk, J. Org. Chem., 1982, 47, 5030-5032).

Die Nachteile aller oben dargestellten Aktivierungsmethoden für Zink liegen darin, dass sie entweder relativ aufwendig und kostspielig oder nur sehr beschränkt anwendbar bzw. wirksam sind, oder zu nicht reproduzierbaren Ausbeuten führen. Das technische Problem einer effizienten und wirtschaftlichen Durchführung weiterer zinkorganischer Reaktionen, die nicht mit den erwähnten Nachteilen behaftet sind, ist somit bisher nicht gelöst.

Überraschenderweise wurde nun gefunden, dass die Behandlung schmelzflüssigen Zinks mit Metallhydriden Zink erzeugt, das in vortrefflicher Weise die eingangs dargelegten Nachteile überwindet und unter Verzicht auf die beschriebenen Aktivierungsmethoden des Standes der Technik direkt und effizient metallorganischen Synthesereaktionen zugeführt werden kann.

Erfindungsgemäß konnte gezeigt werden, dass kommerziell erhältliches metallisches Zink, das mit etwa 2 Gew.-% Metallhydrid (beispielsweise LiH, MgH₂, AlH₃, TiH₂ etc.) oder Metallhydridgemischen bis zum Erreichen des Schmelzpunktes erhitzt wird, nach dem Abkühlen auf Zimmertemperatur und mechanischem Zerspanen, sowohl mit (durch Zink-Kupfer-Paarbildung im Falle der Simmons-Smith-Reaktionen) als auch ohne die oben erwähnten zusätzlichen Aktivierungsverfahren in Simmons-Smith- und Reformatsky-Reaktionen zu deutlich höheren Produktausbeuten führt, als kommerziell erhältliches Zink-Pulver.

Alternativ kann die erfindungsgemäße Behandlung des Zinks auch in schmelzflüssiger Phase erfolgen, indem man die Zinkschmelze mit Metallhydrid oder Metallhydridgemischen beaufschlagt. Demzufolge sind Maßnahmen, die einer raschen Verteilung des/der Metallhydrid(s)e in der flüssigen Zinkmatrix dienen (Rühren, Einblasen von Inertgasen etc.) bei dieser Variante des erfindungsgemäßen Verfahrens vorteilhaft einzusetzen.

Beim Einsatz des nach dem oben beschriebenen Behandlungsverfahren erhaltenen Zinks in Simmons-Smith-Reaktionen, die nach Literaturvorschrift (R. J. Rawson, I. T. Harrison, J. Org. Chem. 1970, 35, 2057-2058) durchgeführt wurden, konnten neue und überraschende Erkenntnisse gewonnen werden.

Als Testreaktion wurde beispielsweise die Umsetzung von Cyclohexen zu Norcaran mit CH₂I₂ als Carbenüberträger gewählt.

Die Ausbeuten an Norcaran liegen bei Einsatz von Zink, das mit Metallhydrid behandelt und mit 10 % Kupfer(I)chlorid aktiviert wurde um 5 % höher als bei Verwendung von kommerziellem Zink, welches ebenfalls mit 10 % Kupfer(I)chlorid aktiviert wurde.

Als Testreaktion für vergleichende Reaktivitätsstudien in Reformatsky-Reaktionen zwischen Zink, das durch Vorbehandlung mit Hydriden erhalten wurde und kommerziell erhältlichem Zink wurden die Ausbeuten bei der Synthese von 3-Hydroxybuttersäureethylester aus Bromessigsäureethylester/Acetaldehyd und die Ausbeuten bei der Synthese von 3-Hydroxyoctansäureethylester aus Bromessigsäureethylester/Hexanal bestimmt (Houben-Weyl, Methoden der organischen Chemie, 1973, Band 13/2a, 818). Beide Zink-Chargen werden ohne weitere gängige Aktivierungsverfahren eingesetzt.

Es zeigte sich bei der Synthese von 3-Hydroxybuttersäureethylester, dass die Produktausbeute bei Verwendung von Metallhydrid-behandeltem Zink um 7 % höher liegt als bei dem Einsatz der kommerziellen Zink-Charge.

Bei der Synthese von 3-Hydroxyoctansäureethylester zeigt sich ein ähnlicher Trend. Die Produktausbeute konnte bei Verwendung von Hydrid-vorbehandeltem Zink durchschnittlich um 4 % gegenüber kommerziellem Zink gesteigert werden.

In allen Untersuchungen gemäß der vorliegenden Erfindung über den Einsatz von Metallhydrid-behandeltem Zink in der Synthese zinkorganischer Reagenzien zeigte sich eindeutig, dass deutlich höhere Produktausbeuten gegenüber der Verwendung kommerzieller Zink-Chargen erzielt werden können. Dies ist um so erstaunlicher wenn man bedenkt, dass in den vorgestellten Reaktivitätsstudien die Metallhydrid-behandelten Zink-Späne mit einer deutlich geringeren zur Verfügung stehenden Metalloberfläche eingesetzt wurden, im Gegensatz zu kommerziell erhältlichem Zink-Pulver. Des weiteren konnte überraschenderweise die allgemein akzeptierte Ansicht, dass nur die Verwendung eines Zink-Kupfer-Paares in Simmons-Smith-Reaktionen zu hohen Ausbeuten an Cyclopropanierungsprodukten führt, eindeutig widerlegt werden. Aus den durchgeführten Untersuchungen ergibt sich erstaunlicherweise genau das gegenteilige Bild. Gerade der Verzicht auf das "aktivierte Metall-Paar" führt zu einer erhöhten Reaktivität, wenn man Metallhydrid-behandeltes Zink einsetzt, wie die Ergebnisse eindrucksvoll widerspiegeln.

Ganz eindeutig zeigte sich, dass die deutlich höhere Aktivität des Metallhydrid-behandelten Zinks gegenüber kommerziellem Zink in der metallorganischen Synthesechemie sich entscheidend auf den Faktor Produktausbeute positiv auswirkt. Es ist offensichtlich, dass sich ein ebenso deutlicher positiver Effekt auf weitere wichtige und entscheidende Reaktionsparameter wie beispielsweise Reaktionszeit, Reaktionstemperatur, Stereo- und Regioselektivität etc. erzielen lässt.

Rein formal betrachtet ist die Simmons-Smith-Reaktion nach Literatur (Houben-Weyl, Methoden der organischen Chemie, 1973, Band 13/2a, 838-852) eine Abfangreaktion der intermediär aus Zn und CH₂X₂ (X = Br, I) gebildeten und auch isolierbaren zinkorganischen Verbindung XCH₂ZnX mit Hilfe eines Olefins unter Bildung von Cyclopropanen und Abspaltung von ZnX₂. Demgemäss bestehen optional weitere Einsatzmöglichkeiten für das erfindungsgemäße Zink auch in der Synthese und Isolierung von Zinkorganylen wie beispielsweise Zinkdialkyl-, Zinkdiaryl-, Zinkalkylaryl-, Zinkalkylhalogenid-, Zinkarylhalogenid-, Zinkdihydrid-, Alkylzinkhydrid-, Arylzinkhydrid-, Zinkdialkylalkoxid-, Zinkdiarylalkoxid-, Alkylzinkalkylalkoxid-, Alkylzinkarylalkoxid-, Arylzinkalkylalkoxid-, Arylzinkarylalkoxid-, Zinkdialkylamid-, Zinkdiarylamid-, Alkylzinkalkylamid-, Alkylzinkarylamid-, Arylzinkalkylamid-, Arylzinkarylamid-Verbindungen.

Die Erfindung wird anhand der folgenden Beispiele exemplarisch erläutert, ohne dass dadurch eine Einschränkung der Anwendbarkeit impliziert werden soll. Die nachfolgend beschriebenen Versuche wurden in Argonatmosphäre durchgeführt. Es wurden luft- und wasserfreie Lösungsmittel verwendet. Toluol und Diethylether wurden über Natrium getrocknet. Die eingesetzten organischen und anorganischen Ausgangssubstanzen wurden ohne weitere Reinigung oder Trocknung eingesetzt.

Es wurden eingesetzt: Dibrommethan (99 %), trans-Crotonsäuremethylester (98 %), Bromessigsäureethylester (98 %), Acetaldehyd (99.5 %) und Hexanal (96 %) der Firma Acros; Cyclohexen (99 %) und 4-Vinylcyclohexen (99 %) der Firma Aldrich; Dijodmethan, Kupfer(I)chlorid (96 %) und Zink-Pulver für die Reaktivitätsstudien der Firma Fisher Scientific (97.2 %). Zur Behandlung von Zink mit Hydriden wurde Zink-Pulver der Firma Grillo-Werke AG (99.95 %) eingesetzt.

### Ausführungsbeispiele:

### Bezugsbeispiel 1:

### Verfahren zur Behandlung metallischen Zinks mit Magnesiumhydrid

294 g handelsübliches Zink-Pulver wurde mit 6 g Magnesiumhydrid-Pulver (Tego® Magnan) für 10 Minuten intensiv miteinander vermischt, dann in einer Stahlkapsel mit einem Druck von 200 Tonnen verpresst und anschließend unter Argon in einem offenen Induktionsofen für 10 Minuten auf 450 °C erwärmt. Nach dem Abkühlen auf Zimmertemperatur wurde der erhaltene massive Zink-Block schmier- und kühlmittelfrei auf eine Größe von durchschnittlich 2 x 10 mm gespannt.

### Beispiel 1:

### Darstellung von Norcaran mit gemäß Bezugsbeispiel 1 vorbehandeltem Zink und Aktivierung mit Kupfer(I)chlorid

8.5 g (0.13 mol) Zink und 1.29 g (0.013 mol) Kupfer(I)chlorid wurden in 40 ml Diethylether suspendiert und 30 Minuten unter Rühren am Rückfluss erhitzt. Anschließend gab man 5.25 ml (0.065 mol) Dijodmethan und 5.05 ml (0.05 mol) Cyclohexen hinzu und erhitzte unter Rühren weitere 24 Stunden am Rückfluss. Dann wurde nach Filtration die organische Phase zweimal mit 20 ml 5 %-iger Salzsäure und dreimal mit 20 ml Wasser extrahiert. Die vereinigten wässrigen Phasen wurden nochmals mit 30 ml Diethylether extrahiert. Die vereinigten organischen Phasen wurden über Na₂SO₄ getrocknet. Ausbeute an Norcaran (GC-Analytik): 71%.

### Beispiel 2:

### Darstellung von Norcaran

8.5 g (0.13 mol) gemäß Bezugsbeispiel 1 vorbehandeltes Zink ohne Aktivierung, 5.25 ml (0.065 mol) Dijodmethan und 5.05 ml (0.05 mol) Cyclohexen wurden in 40 ml Diethylether suspendiert und 24 Stunden unter Rühren am Rückfluss erhitzt. Anschließend wurde nach Filtration die organische Phase zweimal mit 20 ml 5 %-iger Salzsäure und dreimal mit 20 ml Wasser extrahiert. Die vereinigten wässrigen Phasen wurden nochmals mit 30 ml Diethylether extrahiert. Die vereinigten organischen Phasen wurden über Na₂SO₄ getrocknet. Ausbeute an Norcaran (GC-Analytik): 82 %.

### Vergleichsbeispiel 1:

### Analog Beispiel 1 wurde dieses mit handelsüblichem Zink wiederholt. Die Ausbeute betrug 66 %.

Noch verblüffender war die Beobachtung, dass sich analog Beispiel 1 die Produktausbeute an Norcaran um 16 % bei Einsatz des Metallhydrid-behandelten Zinks gegenüber kommerziellem Zink, welches mit 10 % Kupfer(I)chlorid aktiviert wurde, steigern ließ, wenn man auf die Aktivierung mit Kupfer verzichtete. Die nachfolgende Tabelle 1 gibt die Daten der Umsetzung wieder.

**Tabelle 1:**

| | Verhältnis Zn/CH₂I₂/Cyclohexen [mol] | CuCl [mol] | Et₂O [ml] | Ausbeute [GC-%] |
|---|---|---|---|---|
| Beispiel 1 | 0.13/0.065/0.05 | 0.013 | 40 | 71 |
| Vergl. 1 | 0.13/0.065/0.05 | 0.013 | 40 | 66 |
| Beispiel 2 | 0.13/0.065/0.05 | -- | 40 | 82 |

### Beispiel 3:

Darüber hinaus waren um 4 % höhere Ausbeuten an Norcaran bei der Umsetzung von Cyclohexen mit CH₂Br₂ als Carbenquelle bei Einsatz des Metallhydrid-behandelten Zinks gegenüber kommerziellem Zink erzielbar, wenn beide Zink-Chargen mit 10 % Kupfer (I) chlorid aktiviert wurden.

**Tabelle 2:**

| | Verhältnis Zn/CH₂Br₂/Cyclohexen [mol] | CuCl [mol] | Et₂O [ml] | Ausbeute [GC-%] |
|---|---|---|---|---|
| Beispiel 3 | 0.065/0.0325/0.025 | 0.0065 | 20 | 77 |
| Vergl. 2 | 0.065/0.0325/0.025 | 0.0065 | 20 | 73 |

### Beispiele 4, 5, Vergleichsbeispiel 3:

Ergänzend zu den vorgenannten Ausführungsbeispielen wurde analog Beispiel 1 und 2 gefunden, dass bei der Umsetzung von 4-Vinylcyclohexen mit Metallhydrid-behandeltem Zink man zu 71 % Cyclopropanierungsprodukte mit, und zu 72 % Produktausbeute ohne Aktivierung mit 10 % Kupfer(I)chlorid erhielt. Bei der entsprechenden Umsetzung mit kommerziellem Zink und Kupfer-Aktivierung erhielt man lediglich 40 % Ausbeute.

Die folgende Tabelle 3 gibt die erhaltenen Reaktionsdaten wieder.

**Tabelle 3:**

| | Verhältnis Zn/CH₂I₂/4-Vinylcyclohexen [mol] | CuCl [mol] | Gesamtausbeute [GC-%] |
|---|---|---|---|
| Beispiel 4 | 0.065/0.0325/0.025 | 0.0065 | 71 |
| Vergl. 3 | 0.065/0.0325/0.025 | 0.0065 | 40 |
| Beispiel 5 | 0.065/0.0325/0.025 | -- | 72 |

### Beispiel 6, Vergleichsbeispiel 4:

Ein weiterer Aspekt lag darin, dass die Produktausbeute analog Beispiel 1 und 2 bei der Cyclopropanierung von trans-Crotonsäuremethylester bei Verwendung von Zink, das nach dem oben beschriebenen Verfahren behandelt und nicht mit 10 % Kupfer(I)chlorid aktiviert wurde, um 21 % gegenüber kommerziellem Zink mit entsprechender Aktivierung mit Kupfer(I)chlorid gesteigert werden konnte.

Die nachfolgende Tabelle gibt die erhaltenen Reaktionsdaten wieder.

**Tabelle 4:**

| | Verhältnis Zn/CH₂I₂/trans-Crotonsäuremethylester [mol] | CuCl [mol] | Et₂O [ml] | Ausbeute [GC-%] |
|---|---|---|---|---|
| Vergl. 4 | 0.13/0.065/0.05 | 0.013 | 20 | 16 |
| Beispiel 6 | 0.13/0.065/0.05 | -- | 20 | 37 |

### Beispiel 7, Vergleichsbeispiel 5:

### Darstellung von 3-Hydroxybuttersäureethylester

15.28 g (0.234 mol) gemäß Bezugsbeispiel 1 vorbehandeltes Zink wurden in 50 ml Toluol suspendiert, auf 83 °C erwärmt, die Heizquelle anschließend entfernt und tropfenweise eine Mischung aus 22.8 ml (0.206 mol) Bromessigsäureethylester und 10.45 ml (0.187 mol) Acetaldehyd innerhalb von 30 Minuten zugegeben. Nach einer kurzen Induktionsphase beobachtete man starkes Aufschäumen und Erwärmung des Reaktionsansatzes auf 110 °C. Nachdem die gesamte Mischung zugegeben wurde, kühlte man die erhaltene Suspension mit einer Eis-Wasser-Mischung auf 10 °C ab und gab tropfenweise 30 ml 50 %-ige Schwefelsäure so zu, dass die Temperatur der Suspension 35 °C nicht überschritten wurde. Nach der Phasenseparation wurde die organische Phase einmal mit 30 ml 5 %-iger Natriumcarbonatlösung gewaschen und die vereinigten Wasserphasen zweimal mit 30 ml Tetrachlorkohlenstoff extrahiert. Die vereinigten organischen Phasen wurden über Natriumsulfat getrocknet. Nach Filtration und Entfernen aller flüchtigen Bestandteile am Rotationsverdampfer bei 40 °C/5 Torr wurde der erhaltene Rückstand bei 3.5 Torr im Ölpumpenvakuum destilliert. Bei 58-60 °C geht die Fraktion mit 3-Hydroxybuttersäureethylester über. Ausbeute: 7.5 ml (31 %).

Bei Verwendung von kommerziellem Zink-Pulver nach der im Beispiel 7 erläuterten Versuchsvorschrift konnte eine Produktausbeute von 24 % erzielt werden.

**Tabelle 5:**

| | Verhältnis Zn/Bromessigsäureethylester/Hexanal [mol] | Ausbeuten [%] |
|---|---|---|
| Beispiel 7 | 0.117/0.103/0.0935 | 59, 65, 70 |
| Vergl. 5 | 0.117/0.103/0.0935 | 69, 61, 54 |

### Beispiel 8:

### Darstellung von 3-Hydroxyoctansäureethylester

7.64 g (0.117 mol) gemäß Bezugsbeispiel 1 vorbehandeltes Zink wurden in 50 ml Toluol suspendiert, auf 83 °C erwärmt, die Heizquelle anschließend entfernt und tropfenweise eine Mischung aus 11.4 ml (0.103 mol) Bromessigsäureethylester und 11.23 ml (0.0935 mol) Hexanal innerhalb von 30 Minuten zugegeben. Nach einer kurzen Induktionsphase beobachtete man starkes Aufschäumen und Erwärmung des Reaktionsansatzes auf 110 °C. Nachdem die gesamte Mischung zugegeben wurde, kühlte man die erhaltene Suspension mit einer Eis-Wasser-Mischung auf 8 °C ab und gab tropfenweise 15 ml 50 %-ige Schwefelsäure so zu, dass die Temperatur der Suspension 35 °C nicht überschritten wurde. Nach der Phasenseparation wurde die organische Phase zweimal mit 15 ml Wasser gewaschen und über Natriumsulfat getrocknet. Nach Filtration und Entfernen aller flüchtigen Bestandteile am Rotationsverdampfer bei 50 °C/5 Torr wurde der erhaltene Rückstand bei 2.5 Torr im Ölpumpenvakuum destilliert. Bei 88-93 °C ging die Fraktion mit 3-Hydroxyoctansäureethylester über. Ausbeute: 12.25 g (70 %).

## Patentansprüche

1. Verwendung von Metallhydrid-behandeltem Zink in der metallorganischen Synthese.

2. Verwendung nach Anspruch 1, **dadurch gekennzeichnet, dass** man Metallhydrid-behandeltes Zink für die Synthese von Cyclopropan-Derivaten einsetzt.

3. Verwendung nach Anspruch 1, **dadurch gekennzeichnet, dass** man Metallhydrid-behandeltes Zink für Reformatsky- und Reformatsky-analogen Reaktionen einsetzt.

4. Verwendung nach Anspruch 1, **dadurch gekennzeichnet, dass** man Metallhydrid-behandeltes Zink für die Synthese von Zinkorganylen einsetzt.

5. Verwendung nach Anspruch 4, zur Herstellung von Zinkdialkyl-, Zinkdiaryl-, Zinkalkylaryl-, Zinkalkylhalogenid-, Zinkarylhalogenid-, Zinkdihydrid-, Alkylzinkhydrid-, Arylzinkhydrid-, Zinkdialkylalkoxid-, Zinkdiarylalkoxid-, Alkylzinkalkylalkoxid-, Alkylzinkarylalkoxid-, Arylzinkalkylalkoxid-, Arylzinkarylalkoxid-, Zinkdialkylamid-, Zinkdiarylamid-, Alkylzinkalkylamid-, Alkylzinkarylamid-, Arylzinkalkylamid-, Arylzinkarylamid-Verbindungen.
